# EUROPEAN PATENT APPLICATION

(11) **EP 4 691 498 A1**
(43) Date of publication of application: **11.02.2026**
(21) Application number: 24784260.2
(22) Date of filing: 02.04.2024
(51) Int. Cl.: A61K 47/68, A61K 47/55, A61K 47/65, A61K 31/537, A61K 31/4745, A61P 35/00

(54) **ANTIBODY-DRUG CONJUGATE, AND PREPARATION METHOD THEREFOR AND USE THEREOF**

(30) Priority: 07.04.2023 CN 202310367634
(71) Applicant: Chengdu Scimount Pharmatech Co., Ltd., Chengdu, Sichuan 610041 (CN)
(72) Inventor: HUANG, Jinkun, Chengdu, Sichuan 610041 (CN); WU, Chenglong, Chengdu, Sichuan 610041 (CN); KE, Tianyi, Chengdu, Sichuan 610041 (CN); FENG, Chaoyang, Chengdu, Sichuan 610041 (CN); HENG, Xiaojie, Chengdu, Sichuan 610041 (CN); LU, Yue, Chengdu, Sichuan 610041 (CN); YANG, Tingting, Chengdu, Sichuan 610041 (CN)
(74) Representative: Altmann Stößel Dick Patentanwälte PartG mbB
(86) International application number: PCT/CN2024/085455
(87) International publication number: WO 2024/208176

(57) **Abstract**

The present disclosure provides an antibody-drug conjugate, a preparation method, and use thereof, belonging to the field of medicine. The structure of the antibody-drug conjugate is shown in Formula I, in which the antibody Ab is an anti-HER2 antibody, q is an integer from 1 to 20, and D is a drug. The antibody-drug conjugate provided by the present disclosure has low aggregation and low naked antibody, appropriate DAR, exhibits excellent plasma stability and anti-tumor effect, can effectively inhibit tumor recurrence, and has good clinical application prospect.

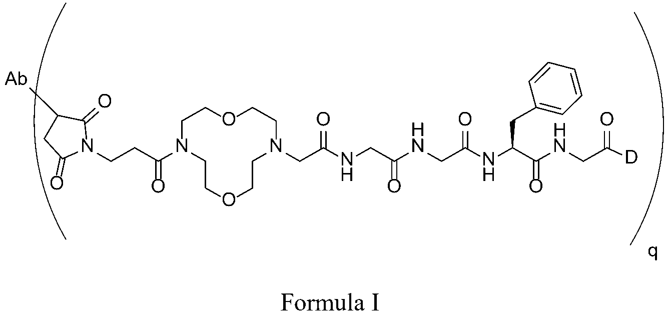

## Description

### CROSS-REFERENCE TO RELATED APPLICATIONS

.

### TECHNICAL FIELD

The present disclosure belongs to the field of medicine, and specifically relates to an antibody-drug conjugate with an anti-HER2 antibody as a targeting moiety, a preparation method, and use thereof.

### BACKGROUND

Antibody-Drug Conjugate (ADC) can selectively deliver drugs to cancer cells and kill them while having less impact on normal cells, ushering in a new era of tumor treatment. A number of ADC drugs have been approved by the FDA for marketing, such as Mylotarg formed by linking a CD33 antibody to calicheamicin, Adcetris formed by linking a CD30 antibody to auristatin E for the treatment of patients with Hodgkin's lymphoma and anaplastic large cell lymphoma, and DS-8201 formed by linking a HER2 antibody to camptothecin derivative Dxd for the treatment of patients with HER2-positive breast cancer, and Sacituzumab govitecan targeting the TROP-2 antigen (also known as epithelial glycoprotein 1, EGP-1) for triple-negative breast cancer.

The drugs contained in the ADCs recognized by the FDA so far mainly target DNA or tubulin. As small molecule anti-tumor compounds, camptothecin derivatives (such as SN-38, Dxd, Dx-8951), which are known to exert anti-tumor effects by inhibiting DNA topoisomerase I, have been confirmed to kill a variety of cancer cells both in vitro and in vivo, showing strong anti-tumor effects. Compounds that exert anti-tumor effects by inhibiting tubulin, such as Eribulin, MMAE, MMAF, and maytansinoid, have been confirmed to kill a variety of cancer cells both in vitro and in vivo, showing strong anti-tumor effects. The structural formulas of the known compounds that can act on DNA or tubulin are as follows:

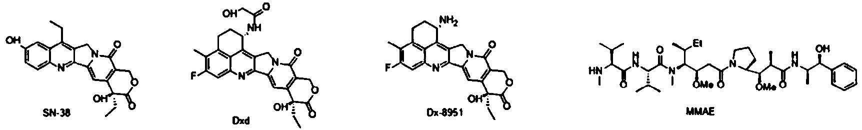

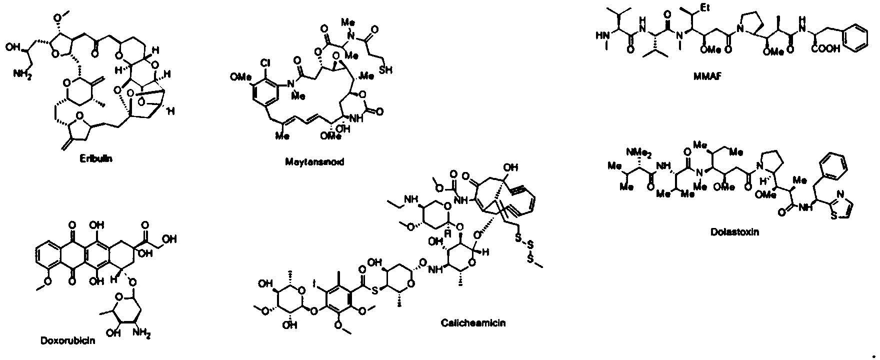

According to the most classic mechanism of action of ADC drugs, antibody-drug conjugates can specifically bind to receptors on the cell surface, and the resulting conjugates are endocytosed by cells, thereby achieving the targeted delivery of drug molecules into cells. Therefore, the concentration of intracellular drugs is directly related to the distribution density of receptors on the cell surface that can be specifically recognized by antibodies. Unfortunately, the density of receptors on the molecular surface that can be recognized by antibodies is usually low, resulting in a lower drug concentration within target cells. To solve this problem, a more commonly used method at present is to increase the drug-antibody ratio (DAR) of ADCs, thereby increasing the number of drugs entering the cell. However, according to a study by Hamblett et al. (Clin Cancer Res. 2004, 10, 7063), increasing the DAR of ADCs usually has the opposite effect to the expected in pharmacokinetic exposure. ADC molecules will undergo severe aggregation and reduced stability, resulting in an increase in small-molecule toxins in the blood and causing toxic and side effects.

Daiichi Sankyo Co., Ltd. has filed a number of patent applications (CN201380053256.2, CN201910768778.X, CN201980061665.4, etc.) since 2013, disclosing a series of ADCs with specific linker- toxin structures, and specifically disclosed ADCs with the following typical structures that show relatively excellent effects. However, their stability and therapeutic effects need to be further improved.

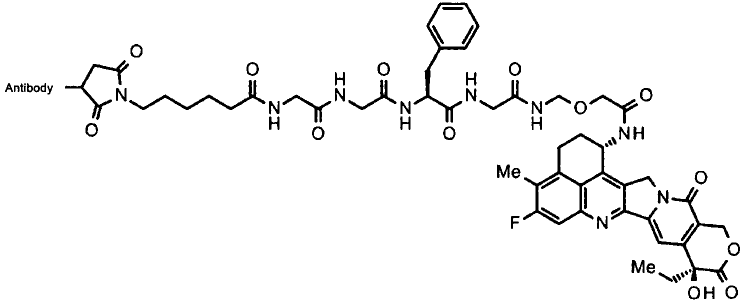

Therefore, it is of great significance to develop a drug conjugate that can significantly improve the aggregation phenomenon, enhance stability and therapeutic effect, and target the lesion site.

### SUMMARY

The objective of the present disclosure is to provide an antibody-drug conjugate with an anti-HER2 antibody as a targeting moiety, a preparation method, and use thereof.

The present disclosure provides an antibody-drug conjugate, or a stereoisomer, an optical isomer, a salt, or a deuterated derivative thereof, and the structure of the antibody-drug conjugate is as shown in Formula I: 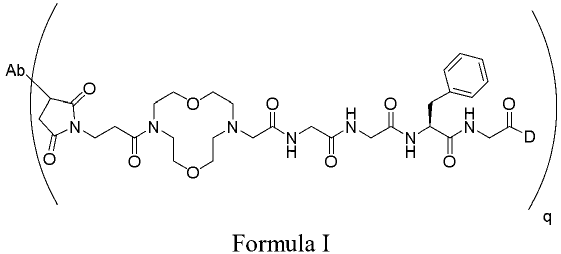
Ab is an anti-HER2 antibody, and the anti-HER2 antibody is inetetamab or trastuzumab;
q is an integer from 1 to 20; and
D is a drug.

Further, D is a cytotoxic drug, a drug for treating autoimmune toxicity or an anti-inflammatory drug;
preferably, D is a drug targeting DNA or a drug targeting tubulin;
more preferably, D is one of the following compounds or derivatives thereof:

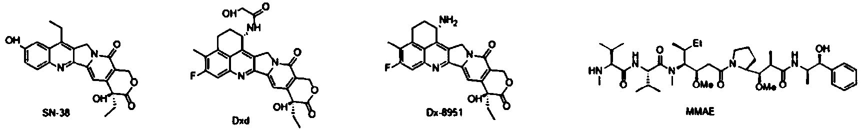

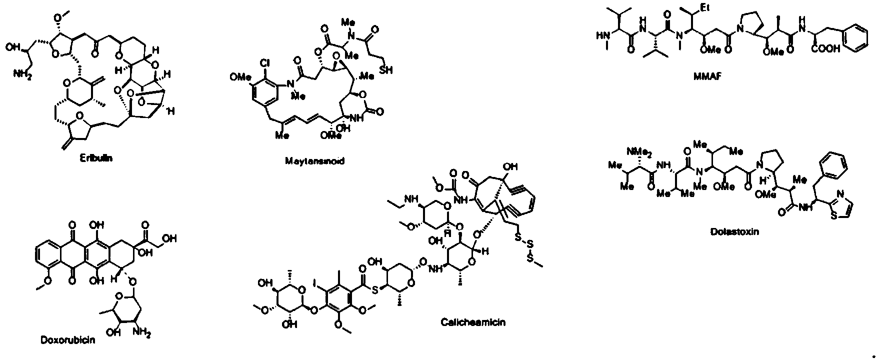

Further, the structure of the antibody-drug conjugate is as shown in Formula II: 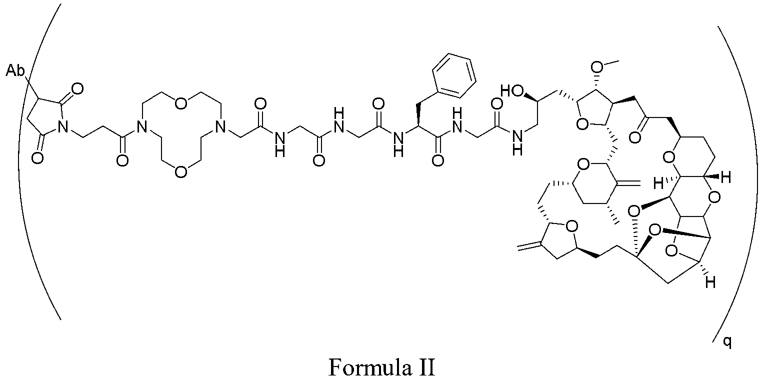
Ab is an anti-HER2 antibody, and the anti-HER2 antibody is inetetamab or trastuzumab; and
q is an integer from 1 to 20.

Further, the heavy chain sequence of the inetetamab is as shown in SEQ ID NO: 1, and the light chain sequence is as shown in SEQ ID NO: 2.

Further, the heavy chain sequence of the trastuzumab is as shown in SEQ ID NO: 3, and the light chain sequence is as shown in SEQ ID NO: 4.

Further, the DAR of the antibody-drug conjugate is 1.00-20.00, preferably 2.0-8.0.

Further, the DAR of the antibody-drug conjugate is 2.0-5.0, preferably 4.08±0.5.

The present disclosure further provides a pharmaceutical preparation for preventing and/or treating tumors, which is prepared by using the above-mentioned antibody-drug conjugate, or a stereoisomer, an optical isomer, a salt, or a deuterated derivative thereof as an active ingredient, together with pharmaceutically acceptable excipients.

Further, the preparation is an oral preparation or an injection preparation.

The present disclosure further provides use of the above-mentioned antibody-drug conjugate, or a stereoisomer, an optical isomer, a salt, or a deuterated derivative thereof in the preparation of a pharmaceutical preparation for preventing and/or treating tumors.

Further, the tumor is selected from lung cancer, urethral cancer, colorectal cancer, prostate adenocarcinoma, ovarian cancer, pancreatic cancer, breast cancer, bladder cancer, gastric cancer, gastrointestinal stromal tumor, cervical cancer, esophageal cancer, squamous cell carcinoma, peritoneal cancer, liver cancer, colon cancer, rectal cancer, colorectal cancer, uterine cancer, salivary gland cancer, kidney cancer, vulvar cancer, thyroid cancer, penile cancer, leukemia, malignant lymphoma, plasmacytoma, myeloma, or sarcoma, and biliary tract tumor.

The present disclosure further provides a method for preparing the above-mentioned antibody-drug conjugate, or a stereoisomer, an optical isomer, a salt, or a deuterated derivative thereof. The method includes the following steps: conjugating an anti-HER2 antibody with Compound III to obtain the antibody-drug conjugate; the structure of Compound III is as follows:

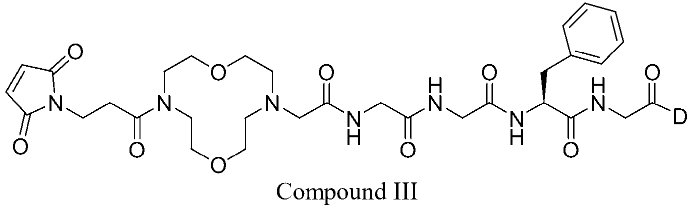

D is the above-mentioned drug.

Definition of terms used in the present disclosure: Unless otherwise specified, the initial definition of a group or term provided herein applies to that group or term throughout the specification; for terms not specifically defined herein, meanings that can be attributed to them by those skilled in the art should be given based on the disclosure content and context.

"Targeting moiety-drug conjugate" refers to a conjugate formed by conjugating a drug with a targeting moiety that has the function of targeting and binding to the lesion site through a linker. For example, "Antibody-Drug Conjugate" (ADC) is a type of targeting moiety-drug conjugate, and its targeting moiety is an antibody or antibody fragment.

"Drug-linker assembly unit" refers to a product obtained by connecting the linker and the drug in the aforementioned targeting moiety-drug conjugate, which is an intermediate for preparing the targeting moiety-drug conjugate. By conjugating it with an antibody, the antibody-drug conjugate can be obtained.

In the present disclosure, "DAR" represents the average number of drug-linker assembly units conjugated to an antibody in the antibody-drug conjugate, which is equivalent to the average value of q. The DAR may not be an integer.

"Deuterated derivative" refers to a compound obtained by replacing one or more hydrogen atoms in the compound with deuterium.

"Linker", also known as a "connector", is a substance used to link a compound with therapeutic effects to a targeting moiety that has the function of targeting and binding to the lesion site. "Linker fragment" refers to the part of the linker that has a connecting function.

According to the mechanism of intracellular drug release, "linkers" can be divided into two categories: non-cleavable linkers and cleavable linkers.

For targeting moiety-drug conjugates (such as antibody-drug conjugates) containing non-cleavable linkers, the drug release mechanism can be as follows: after the conjugate binds to the antigen and is endocytosed by the cell, the antibody is enzymatically degraded in the lysosome to release the active small molecule composed of the drug with linker and amino acid residue in antibody.

In contrast, targeting moiety-drug conjugates (such as antibody-drug conjugates) containing cleavable linkers can be cleaved in the target cell to release the drug (such as the small molecule drug itself). Cleavable linkers can be divided into two main categories: chemically unstable linkers and enzymatically unstable linkers. Chemically unstable linkers can be selectively cleaved due to different plasma and cytoplasm properties, such as pH value, glutathione concentration, etc. Enzymatically unstable linkers, such as peptide linkers, can be effectively cleaved by proteases in the lysosome, such as cathepsin and plasmin. Peptide linkers are considered to be very stable in plasma.

The linker is a core part of targeting moiety-drug conjugates (such as antibody-drug conjugates), and can greatly affect the pharmacokinetics, therapeutic index and efficacy of targeting moiety-drug conjugates (such as antibody-drug conjugates).

The present disclosure creatively adds the 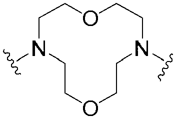 structure shown in the linker, so that the linker not only has the function of a traditional linker, but also can significantly improve the safety, stability, effectiveness, and controllability of the prepared antibody-drug conjugate, and can effectively promote the clinical application of antibody-drug conjugate drugs.

The antibody-drug conjugate provided by the present disclosure includes an anti-HER2 antibody and 1 to 20 covalently linked drug-linker assembly units, in which the drug-linker assembly units can be linked to the thiols generated by the reduction of interchain disulfide bonds in the antibody and/or each linker assembly unit is linked to the thiol from a cysteine residue.

For the convenience of connection, the drug-linker assembly unit is usually constructed before being connected to the anti-HER2 antibody. However, the order of the construction can also be changed. For example, an assembly unit with a protecting group is first connected to the anti-HER2 antibody, and after connecting to the anti-HER2 antibody, the protecting group is removed and other drug units are added.

The antibody-drug conjugate provided by the present disclosure can target tumor cells expressing HER2 receptor and bind to HER2 receptor on the cell surface. The conjugate can enter the cell through endocytosis, and the drug is released into the cell in an active form to exert its efficacy, or the drug is released outside the cell, and penetrates into the cell to exert its efficacy.

The present disclosure has the following beneficial effects:

By introducing the structure 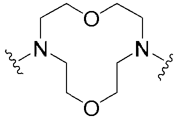 shown in the linker, the aggregation and naked antibody in the antibody-drug conjugate formed by connecting the linker with the drug unit and the anti-HER2 antibody can be reduced, the DAR of the antibody-drug conjugate can be maintained within an appropriate range, the plasma stability of the antibody-drug conjugate can be improved, and the efficacy can be enhanced. The antibody-drug conjugate finally prepared has low aggregation and low percentage of naked antibody, appropriate DAR, exhibits excellent plasma stability and anti-tumor effect, can effectively inhibit tumor recurrence, and has good clinical application prospect.

Obviously, according to the above content of the present disclosure, in accordance with ordinary technical knowledge and conventional means in this field, without departing from the above basic technical ideas of the present disclosure, various other forms of modifications, substitutions or changes can be made.

The above content of the present disclosure will be further described in detail below through specific implementations in the form of embodiments. However, this should not be understood as limiting the scope of the above subject matter of the present disclosure to the following embodiments. All technologies implemented based on the above content of the present disclosure belong to the scope of the present disclosure.

### BRIEF DESCRIPTION OF DRAWINGS

FIG. 1. Stability of ADC in mouse, dog, monkey, and human plasma.
FIG. 2. Anti-tumor effects of ADC in tumor models of ovarian cancer SKOV-3, gastric cancer NCI-N87, pancreatic cancer Capan-1, breast cancer JIMT-1, and breast cancer HCC1954.
FIG. 3. Effect of ADC on sustained inhibition of tumors in vivo.

### DETAILED DESCRIPTION OF THE EMBODIMENTS

The raw materials and equipment used in the present disclosure are all known products obtained by purchasing commercially available products.

### Example 1: preparation of antibody-drug conjugate (SMP-656)

### I. Preparation of intermediate A

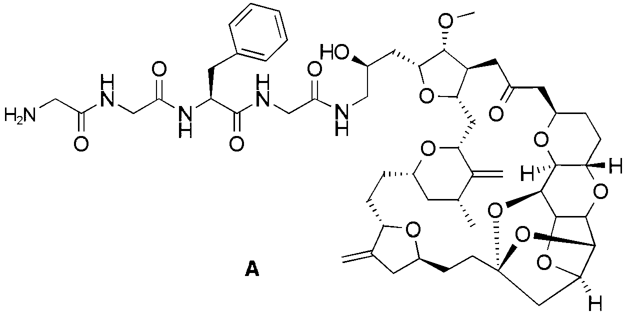

### Step 1: preparation of compound A-2

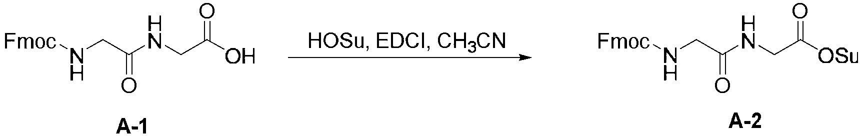

Add A-1 (20.0 g, 56.49 mmol) and acetonitrile (200 mL) into a 500 mL reaction flask, and stir them at room temperature. After complete clarification, add 1-(3-dimethylaminopropyl)-3-ethylcarbodiimide hydrochloride (11.0 g, 57.38 mmol) and N-hydroxysuccinimide (7.0 g, 60.82 mmol), and continue stirring them at room temperature for 12 h. The thin-layer chromatograpgy (TLC) shows that the reaction is complete. Filter the reaction system, and vacuum-dry the obtained solid to obtain compound A-2 with a weight of 22.0 g and a yield of 86%.

### Step 2: preparation of compound A-3

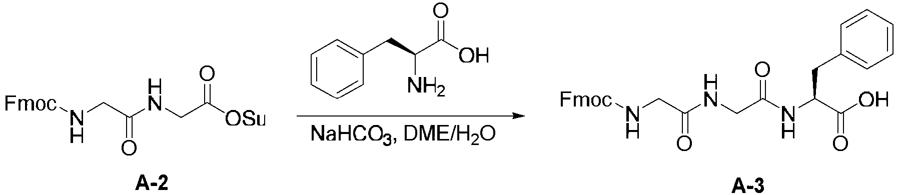

Add L-phenylalanine (8.0 g, 48.48 mmol), sodium bicarbonate (8.0 g, 95.24 mmol), and water (200 mL) to a 500 mL reaction flask, and stir them at room temperature. After complete clarification, dissolve compound A-2 (22.0 g, 48.78 mmol) in ethylene glycol dimethyl ether (50 mL), and slowly add dropwise to the above reaction solution. After the addition is completed, continue stirring them at room temperature for 12 h. TLC shows that the reaction is complete. Concentrate under reduced pressure to remove ethylene glycol dimethyl ether, and add the remaining reaction solution dropwise to 0.5 M hydrochloric acid aqueous solution (500 mL). A large amount of solid is precipitated. Filter, and vacuum-dry the obtained solid to obtain compound A-3 with a weight of 15.0 g and a yield of 61%.

¹HNMR (400 MHz, CDCl3) 12.51 (s, 1H), 9.04 (s, 1H), 8.31 (s, 1H), 7.95 (s, 1H), 7.90(d, J=8.0 Hz, 2H), 7.56(d, J=7.8 Hz, 2H), 7.38-7.28 (m, 4H), 7.19-7.14(m, 5H), 4.85(t, J=8.2 Hz, 1H), 4.71(d, J=8.2 Hz, 2H), 4.39(t, J=8.4 Hz, 1H), 4.10-3.83(m, 4H), 3.12(d, J=9.6 Hz, 1H), 2.85(d, J=9.6 Hz, 1H).

### Step 3: preparation of compound A-4

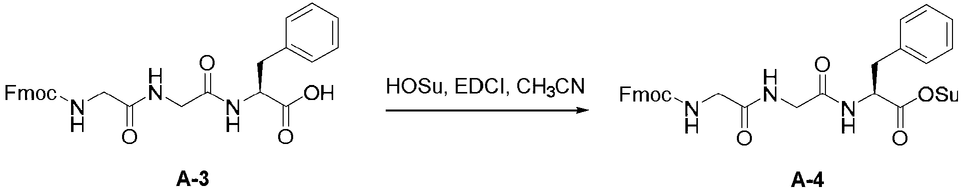

Add A-3 (15.0 g, 29.94 mmol), acetonitrile (200 mL), 1-(3-dimethylaminopropyl)-3-ethylcarbodiimide hydrochloride (6 g, 31.30 mmol) and N-hydroxysuccinimide (4 g, 34.78 mmol) sequentially into a 500 mL reaction flask, and stir them at room temperature to react for 12 h. TLC shows that the reaction is complete. Filter the reaction system, and vacuum-dry the obtained solid to obtain compound A-4 with a weight of 13.0 g and a yield of 73%.

### Step 4: preparation of compound A-5

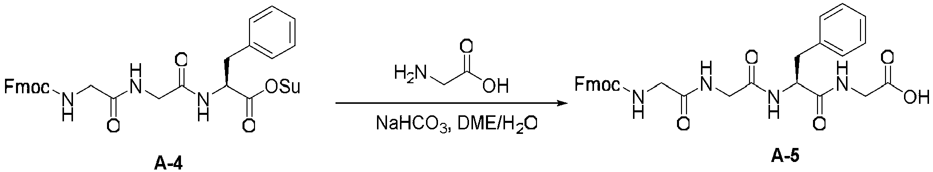

Add glycine (3.0 g, 40.00 mmol), sodium bicarbonate (6.7 g, 79.76 mmol), and water (150 mL) into a 500 mL reaction flask, and stir them at room temperature. After complete clarification, dissolve compound A-4 (13.0 g, 21.74 mmol) in ethylene glycol dimethyl ether (40 mL), and slowly add dropwise to the above reaction solution. After the addition is completed, continue stirring them at room temperature for 12 h. TLC shows that the reaction is complete. Concentrate under reduced pressure to remove the organic solvent, and add the remaining reaction solution dropwise to 0.5 M hydrochloric acid aqueous solution (300 mL). A large amount of solid is precipitated. Filter, and vacuum-dry the obtained solid to obtain compound A-5 with a weight of 10.0 g and a yield of 83%.

¹HNMR (400 MHz, CDCl3) 13.01 (s, 1H), 9.01 (s, 1H), 8.27 (s, 1H), 7.98 (s, 1H), 7.89(d, J=8.0 Hz, 2H), 7.54(d, J=7.8 Hz, 2H), 7.34-7.23 (m, 4H), 7.19-7.14(m, 5H), 4.75(t, J=8.2 Hz, 1H), 4.61(d, J=8.2 Hz, 2H), 4.30(t, J=8.4 Hz, 1H), 4.04-3.83(m, 6H), 3.10(d, J=9.6 Hz, 1H), 2.75(d, J=9.6 Hz, 1H).

### Step 5: preparation of compound A-6

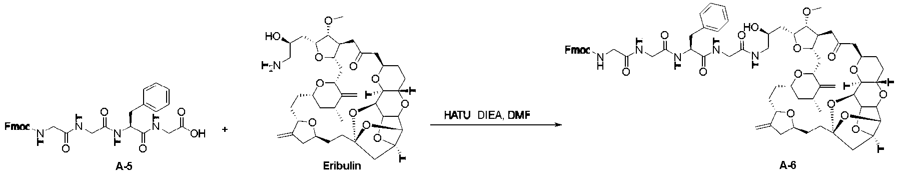

Add A-5 (200.0 mg, 0.36 mmol), O-(7-azabenzotriazol-1-yl)-N,N,N',N'-tetramethyluronium hexafluorophosphate (163.0 mg, 0.43 mmol), N,N-diisopropylethylamine (66.9 mg, 0.52 mmol), and N,N-dimethylformamide (5 mL) sequentially into a 25 mL reaction flask. After stirring them at room temperature for 5 min, add eribulin (263.1 mg, 0.36 mmol). Continue stirring them at room temperature for 10 min. TLC shows that the reaction is complete. Concentrate under reduced pressure to remove N,N-dimethylformamide, and purify the residue by thin-layer chromatography (dichloromethane:methanol = 10:1) to obtain compound A-6 with a weight of 300.0 mg and a yield of 66%.

### Step 6: preparation of compound A

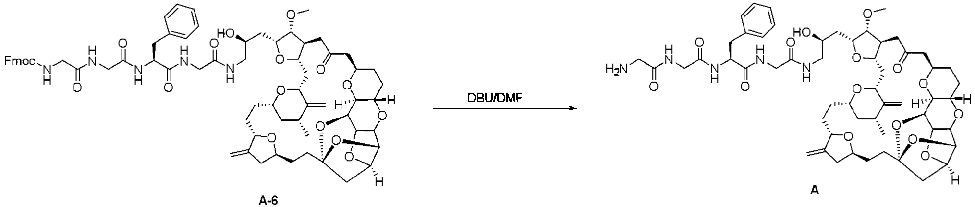

Add N,N-dimethylformamide (2 mL), A-6 (20.0 mg, 0.016 mmol), and 1,8-diazabicyclo[5.4.0]undec-7-ene (5.0 mg, 0.032 mmol) sequentially into a 25 mL reaction flask. Stir them at room temperature for 30 min. TLC shows that the reaction is complete. Concentrate under reduced pressure to remove N,N-dimethylformamide, and purify the residue by preparative-scale high performance liquid chromatography (HPLC) to obtain compound A with a weight of 10.0 mg and a yield of 60%.

¹HNMR (400 MHz, DMSO-*d*₆) δ 8.48 (t, *J =* 5.5 Hz, 1H), 8.31 (dd, *J* = 10.1, 4.6 Hz, 2H), 7.98 (s, 3H), 7.74 (t, *J =* 5.6 Hz, 1H), 7.30 - 7.21 (m, 4H), 7.22 - 7.15 (m, 1H), 5.02 (d, *J =* 22.6 Hz, 2H), 4.79 (d, *J =* 29.3 Hz, 2H), 4.63 (dd, *J =* 5.7, 3.7 Hz, 1H), 4.59 - 4.50 (m, 2H), 4.24 (t, *J* = 13.5 Hz, 1H), 4.21 - 4.12 (m, 1H), 4.12 - 4.06 (m, 3H), 4.02 (s, 1H), 3.86 (dd, *J =* 16.8, 5.7 Hz, 1H), 3.68 (dd, *J* = 16.7, 5.5 Hz, 4H), 3.54 (dd, *J =* 19.0, 7.3 Hz, 12H), 3.11 (dd, *J* = 16.3, 11.0 Hz, 1H), 3.07 - 2.99 (m, 2H), 2.84 (d, *J* = 9.6 Hz, 1H), 2.80 - 2.65 (m, 3H), 2.62 - 2.54 (m, 1H), 2.24 (ddd, *J* = 48.1, 23.9, 9.6 Hz, 6H), 1.98 (dd, *J* = 29.5, 15.1 Hz, 6H), 1.73 - 1.57 (m, 5H), 1.55 - 1.40 (m, 2H), 1.37 - 1.25 (m, 3H), 1.22 - 1.13 (m, 1H), 1.03 (d, *J=* 6.4 Hz, 3H), 1.00 - 0.89 (m, 1H).

### II. Preparation of compound SMP-93566

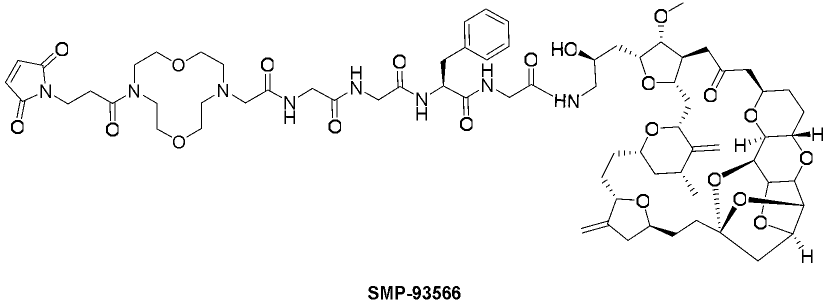

### Step 1: preparation of compound SMP-93566-2

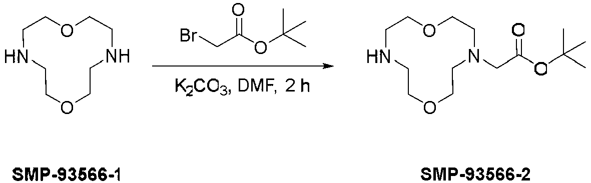

Add intermediate SMP-93566-1 (1.0 g, 5.75 mmol), tert-butyl bromoacetate (0.9 g, 4.60 mmol), potassium carbonate (0.6 g, 4.60 mmol), and N,N-dimethylformamide (20 mL) sequentially into a 100 mL reaction flask. React at room temperature for 2 h. The liquid chromatography-mass spectrometry (LC-MS) shows that most of the product has the molecular weight of the target compound. Filter the reaction solution, concentrate the filtrate under reduced pressure to obtain a crude product. Purify the crude product by column chromatography (dichloromethane:methanol = 12:1) to obtain compound SMP-93566-2 with a weight of 0.2 g and a yield of 15.1%.

MS (ESI) m/z: 289[M+H]⁺.

¹HNMR (400 MHz, CDCl3) δ5.91 (s, 1H), 3.40-3.49(m, 8H), 323 (s, 2H), 2.54(m, 4H), 2.70(m, 4H),1.33 (s, 9H).

### Step 2: preparation of compound SMP-93566-3

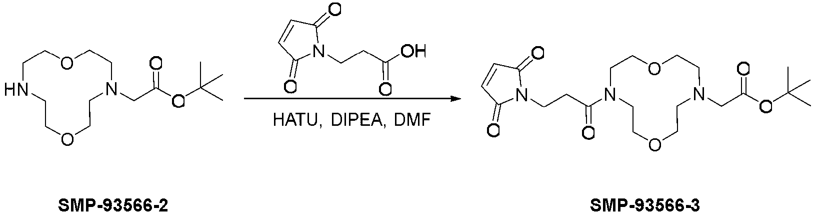

Add intermediate SMP-93566-2 (200.0 mg, 0.69 mmol), maleimidopropionic acid (140.8 mg, 0.83 mmol), N,N-dimethylformamide (5 mL), O-(7-azabenzotriazol-1-yl)-N,N,N',N'-tetramethyluronium hexafluorophosphate (315.4 mg, 0.83 mmol), and N,N-diisopropylethylamine (134.2 mg, 1.04 mmol) sequentially into a 10 mL reaction flask. React at room temperature for 30 min. LC-MS shows that most of the product has the molecular weight of the target compound. Concentrate the reaction solution under reduced pressure to obtain a crude product. Purify the crude product by preparative-scale HPLC to obtain compound SMP-93566-3 with a weight of 220.0 mg and a yield of 72.6%.

MS (ESI) *m*/*z:* 440[M+H]⁺.

### Step 3: preparation of compound SMP-93566-4

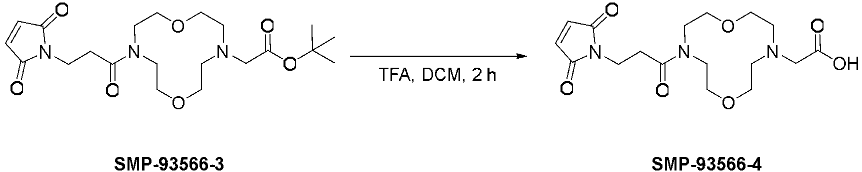

Add SMP-93566-3 (220.0 mg, 0.50 mmol), dichloromethane (5 mL), and trifluoroacetic acid (5 mL) sequentially into a 25 mL reaction flask. React at room temperature for 2 h. LC-MS shows that most of the product has the molecular weight of the target compound. Concentrate the reaction solution under reduced pressure to obtain a crude product. Dissolve the crude product with a small amount of acetonitrile, and purify by preparative-scale HPLC to obtain compound SMP-93566-4 with a weight of 90.0 mg and a yield of 47.0%.

MS (ESI) m/z: 384[M+H]⁺.

### Step 4: preparation of compound SMP-93566

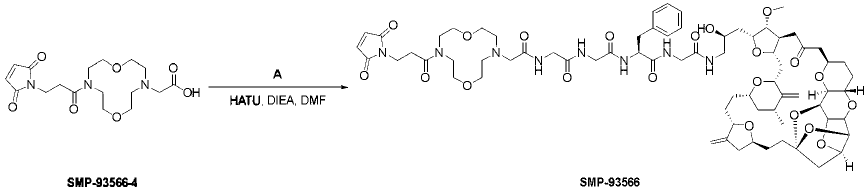

Add SMP-93566-4 (8.8 mg, 0.023 mmol), intermediate A (20 mg, 0.019 mmol), N,N-dimethylformamide (2 mL), O-(7-azabenzotriazol-1-yl)-N,N,N',N'-tetramethyluronium hexafluorophosphate (8.7 mg, 0.023 mmol), and N,N-diisopropylethylamine (3.7 mg, 0.029 mmol) sequentially into a 10 mL reaction flask. React at room temperature for 20 min. LC-MS shows that most of the product has the molecular weight of the target compound. Concentrate the reaction solution under reduced pressure to obtain a crude product. Purify the crude product by preparative-scale HPLC to obtain compound SMP-93566 with a weight of 18.0 mg and a yield of 67.0%.

MS (ESI) *m*/*z:* 1414[M+H]⁺, 707[M/2+H]⁺.

¹H NMR (400 MHz, DMSO-*d*₆) δ 8.89 (s, 1H), 8.29 (t, *J* = 5.6 Hz, 1H), 8.19 (d, *J* = 8.2 Hz, 1H), 8.12 *(t, J =* 5.6 Hz, 1H), 7.95 (s, 1H), 7.72 (s, 1H), 7.31 - 7.09 (m, 6H), 6.98 (s, 2H), 5.02 (d, *J = 21.4* Hz, 2H), 4.82 (s, 1H), 4.75 (s, 1H), 4.63 (s, 1H), 4.58 - 4.48 (m, 2H), 4.28 - 4.06 (m, 10H), 3.87 - 3.47 (m, 30H), 3.16 - 2.99 (m, 5H), 2.85 - 2.64 (m, 6H), 2.62 - 2.53 (m, 2H), 2.34 - 2.16 (m, 6H), 2.01 - 1.87 (m, 6H), 1.73 - 1.58 (m, 6H), 1.31 (s, 3H), 1.19 (m, 1H), 1.03 (d, *J=* 6.3 Hz, 3H), 1.00 - 0.91 (m, 1H).

### III. Preparation of antibody-drug conjugate (SMP-656)

Conjugation method: The disulfide bonds on the antibody are used for drug conjugation to generate the antibody-drug conjugate. First, the antibody is concentrated into a solution with a concentration of 20 mg/mL. Take 0.1 mL of the antibody into a 1.5 mL centrifuge tube, add PBS (pH 7.4)/DTPA solution (293 µL), then add 5 mM TCEP aqueous solution (6.4 µL, 2.4 equivalents relative to one molecule of antibody), and incubate at 25°C for 2 h to reduce the interchain disulfide bonds in the hinge region of the antibody to thiols. Then, add 10% medical DMSO containing 10 mM payload (13.3 µL, 10 equivalents relative to one molecule of antibody) to the above solution, and shake them at 25°C (speed: 400 rpm) for 2 h to connect the drug-linker assembly unit with the antibody to obtain the antibody-drug conjugate.

Compound SMP-93566 is used as the drug-linker assembly unit and conjugated with the anti-HER2 antibody using the aforementioned conjugation method to obtain the corresponding antibody-drug conjugate, named SMP-656.

Concentration of the antibody-drug conjugate: Put the antibody-drug conjugate into a 5,000K (Millipore Co.) ultrafiltration tube, and perform centrifugation at 2°C using a high-speed refrigerated centrifuge (GENESPEED 1,580R) (3,500G for 10 min).

The sequence of the anti-HER2 antibody used in this example is as follows:
Inetetamab
Heavy chain (SEQ ID NO: 1): 
EVQLVESGGG LVQPGGSLRL SCAASGFNIK DTYIHWVRQA PGKGLEWVAR IY
PTNGYTRYADSVKGRFTI SADTSKNTAY LQMNSLRAED TAVYYCSRWG GDGFYAMD
YW GQGTLVTVSSASTKGPSVFP LAPSSKSTSG GTAALGCLVK DYFPEPVTVS WNSGA
LTSGV HTFPAVLQSSGLYSLSSVVT VPSSSLGTQT YICNVNHKPS NTKVDKKVEP KSCD
KTHTCP PCPAPELLGGPSVFLFPPKP KDTLMISRTP EVTCVVVDVS HEDPEVKFNW YV
DGVEVHNA KTKPREEQYNSTYRVVSVLT VLHQDWLNGK EYKCKVSNKA LPAPIEKTI
S KAKGQPREPQ VYTLPPSREEMTKNQVSLTC LVKGFYPSDI AVEWESNGQP ENNYKT
TPPV LDSDGSFFLY SKLTVDKSRWQQGNVFSCSV MHEALHNHYT QKSLSLSPG;
Light chain (SEQ ID NO: 2):

DIQMTQSPSS LSASVGDRVT ITCRASQDVN TAVAWYQQKP GKAPKLLIYS ASF
LYSGVPSRFSGSRSGTD FTLTISSLQP EDFATYYCQQ HYTTPPTFGQ GTKVEIKRTV AAP
SVFIFPPSDEQLKSGTA SVVCLLNNFY PREAKVQWKV DNALQSGNSQ ESVTEQDSKD
STYSLSSTLTLSKADYEKHK VYACEVTHQG LSSPVTKSFN RGEC.

The following is the preparation method of the control ADC sample.

### Comparative example 1: preparation of ADC corresponding to DS-8201 (DS-8201a)

### I. Preparation of compound DS-8201

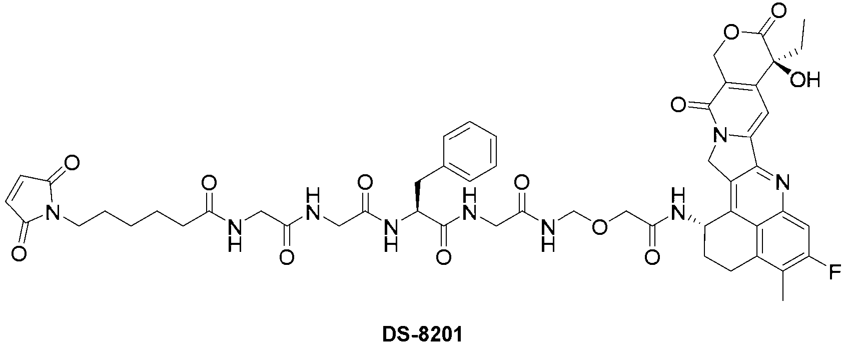

### Step 1: preparation of compound DS-8201

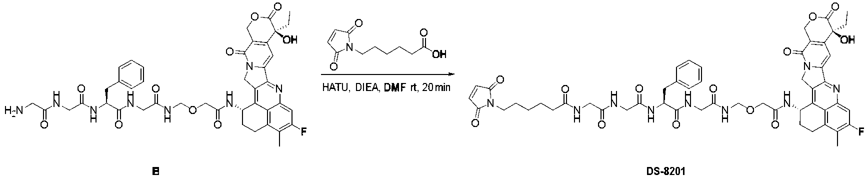

Add intermediate B (50.0 mg, 0.06 mmol), 6-maleimidocaproic acid (12.6 mg, 0.06 mmol), and N,N-dimethylformamide (2 mL) sequentially into a 10 mL reaction flask. After complete dissolution, add O-(7-azabenzotriazol-1-yl)-N,N,N',N'-tetramethyluronium hexafluorophosphate (22.2 mg, 0.06 mmol) and N,N-diisopropylethylamine (23.2 mg, 0.18 mmol) sequentially. Stir them at room temperature for 20 min. TLC shows that the reaction is complete. Purify the reaction solution by preparative-scale HPLC to obtain compound DS-8201 with a weight of 35 mg and a yield of 56.4%.

MS (ESI) *m*/*z :* 1034[M+H]⁺.

### II. Preparation of DS-8201a

Conjugation method: The disulfide bonds on the antibody are used for drug conjugation to generate the antibody-drug conjugate. First, the antibody is concentrated into a solution with a concentration of 20 mg/mL. Take 0.1 mL of the antibody into a 1.5 mL centrifuge tube, add PBS (pH 7.4)/DTPA solution (293 µL), then add 5 mM TCEP aqueous solution (16.0 µL, 6.0 equivalents relative to one molecule of antibody), and incubate at 25°C for 2 h to reduce the interchain disulfide bonds in the hinge region of the antibody to thiols. Then, add 10% medical DMSO containing 10 mM payload (13.3 µL, 10 equivalents relative to one molecule of antibody) to the above solution, and shake them at 25°C (speed: 400 rpm) for 2 h to connect the drug-linker assembly unit with the antibody to obtain the antibody-drug conjugate.

Compound DS-8201 is used as a drug-linker assembly unit and conjugated with the anti-HER2 antibody using the aforementioned conjugation method to obtain the corresponding antibody-drug conjugate, named DS-8201a.

Concentration of the antibody-drug conjugate: Put the antibody-drug conjugate into a 5,000K (Millipore Co.) ultrafiltration tube, and perform centrifugation at 2°C using a high-speed refrigerated centrifuge (GENESPEED 1,580R) (3,500G for 10 min).

The sequence of the anti-HER2 antibody used in this comparative example is as follows:
Trastuzumab
Heavy chain (SEQ ID NO: 3):

EVQLVESGGG LVQPGGSLRL SCAASGFNIK DTYIHWVRQA PGKGLEWVAR IY
PTNGYTRYADSVKGRFTI SADTSKNTAY LQMNSLRAED TAVYYCSRWG GDGFYAMD
YW GQGTLVTVSSASTKGPSVFP LAPSSKSTSG GTAALGCLVK DYFPEPVTVS WNSGA
LTSGV HTFPAVLQSSGLYSLSSVVT VPSSSLGTQT YICNVNHKPS NTKVDKKVEP KSCD
KTHTCP PCPAPELLGGPSVFLFPPKP KDTLMISRTP EVTCVVVDVS HEDPEVKFNW YV
DGVEVHNA KTKPREEQYNSTYRVVSVLT VLHQDWLNGK EYKCKVSNKA LPAPIEKTI
S KAKGQPREPQ VYTLPPSREEMTKNQVSLTC LVKGFYPSDI AVEWESNGQP ENNYKT
TPPV LDSDGSFFLY SKLTVDKSRWQQGNVFSCSV MHEALHNHYT QKSLSLSPG;
Light chain (SEQ ID NO: 4):
DIQMTQSPSS LSASVGDRVT ITCRASQDVN TAVAWYQQKP GKAPKLLIYS ASF
LYSGVPSRFSGSRSGTD FTLTISSLQP EDFATYYCQQ HYTTPPTFGQ GTKVEIKRTV AAP
SVFIFPPSDEQLKSGTA SVVCLLNNFY PREAKVQWKV DNALQSGNSQ ESVTEQDSKD

STYSLSSTLTLSKADYEKHK VYACEVTHQG LSSPVTKSFN RGEC.

### Comparative example 2: preparation of ADC corresponding to SMP-79786 (SMP-79786-X)

### I. Preparation of compound SMP-79786

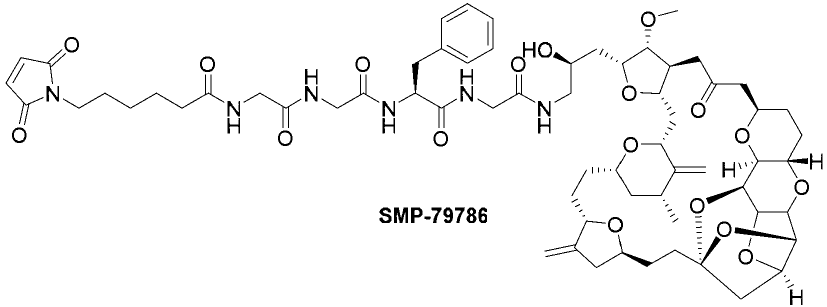

### Step 1: preparation of compound SMP-79786

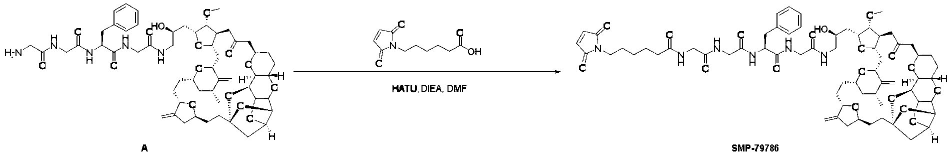

Add 6-maleimidocaproic acid (15.0 mg, 0.071 mmol) and solvent N,N-dimethylformamide (5 mL) into a 25 mL reaction flask. Add O-(7-azabenzotriazol-1-yl)-N,N,N',N'-tetramethyluronium hexafluorophosphate (27.0 mg, 0.071 mmol) and N,N-diisopropylethylamine (11.4 mg, 0.088 mmol) sequentially. After reacting at room temperature for 5 min, add intermediate A (62.0 mg, 0.059 mmol) and react at room temperature for 25 min. TLC shows that the reaction is complete. Concentrate under reduced pressure, and purify the residue by preparative-scale HPLC to obtain compound SMP-79786 with a weight of 48.0 mg and a yield of 65.5%.

MS (ESI) *m*/*z* : 1241 [M+H]⁺.

### II. Preparation of SMP-79786-X

Conjugation method: The disulfide bonds on the antibody are used for drug conjugation to generate the antibody-drug conjugate. First, the antibody is concentrated into a solution with a concentration of 20 mg/mL. Take 0.1 mL of the antibody into a 1.5 mL centrifuge tube, add PBS (pH 7.4)/DTPA solution (293 µL), then add 5 mM TCEP aqueous solution (16.0 µL, 6.0 equivalents relative to one molecule of antibody), and incubate at 25°C for 2 h to reduce the interchain disulfide bonds in the hinge region of the antibody to thiols. Then, add 10% medical DMSO containing 10 mM payload (13.3 µL, 10 equivalents relative to one molecule of antibody) to the above solution, and shake them at 25°C (speed: 400 rpm) for 2 h to connect the drug-linker assembly unit with the antibody to obtain the antibody-drug conjugate.

Compound SMP-79786 is used as the drug-linker assembly unit and conjugated with the anti-HER2 antibody using the aforementioned conjugation method to obtain the corresponding antibody-drug conjugate, named SMP-79786-X.

Concentration of the antibody-drug conjugate: Put the antibody-drug conjugate into a 5,000K (Millipore Co.) ultrafiltration tube, and perform centrifugation at 2°C using a high-speed refrigerated centrifuge (GENESPEED 1,580R) (3,500G for 10 min).

The sequence of the anti-HER2 antibody used in this comparative example is as follows:
Inetetamab
Heavy chain (SEQ ID NO: 1): 
EVQLVESGGG LVQPGGSLRL SCAASGFNIK DTYIHWVRQA PGKGLEWVAR IY
PTNGYTRYADSVKGRFTI SADTSKNTAY LQMNSLRAED TAVYYCSRWG GDGFYAMD
YW GQGTLVTVSSASTKGPSVFP LAPSSKSTSG GTAALGCLVK DYFPEPVTVS WNSGA
LTSGV HTFPAVLQSSGLYSLSSVVT VPSSSLGTQT YICNVNHKPS NTKVDKKVEP KSCD
KTHTCP PCPAPELLGGPSVFLFPPKP KDTLMISRTP EVTCVVVDVS HEDPEVKFNW YV
DGVEVHNA KTKPREEQYNSTYRVVSVLT VLHQDWLNGK EYKCKVSNKA LPAPIEKTI
S KAKGQPREPQ VYTLPPSREEMTKNQVSLTC LVKGFYPSDI AVEWESNGQP ENNYKT
TPPV LDSDGSFFLY SKLTVDKSRWQQGNVFSCSV MHEALHNHYT QKSLSLSPG;
Light chain (SEQ ID NO: 2):

DIQMTQSPSS LSASVGDRVT ITCRASQDVN TAVAWYQQKP GKAPKLLIYS ASF
LYSGVPSRFSGSRSGTD FTLTISSLQP EDFATYYCQQ HYTTPPTFGQ GTKVEIKRTV AAP
SVFIFPPSDEQLKSGTA SVVCLLNNFY PREAKVQWKV DNALQSGNSQ ESVTEQDSKD
STYSLSSTLTLSKADYEKHK VYACEVTHQG LSSPVTKSFN RGEC.

The beneficial effects of the present disclosure were proved by the following test examples.

### Test example 1: quality characterization of antibody-drug conjugates

### 1. Test method:

Size exclusion high performance liquid chromatography (SE-HPLC) was used to detect the aggregation (high-molecular-weight components) of the antibody-drug conjugate. A Biocore SEC column (7.8×300 mm, 5 µm) was used, and the mobile phase was 50 mM phosphate buffer (pH 6.8) containing 300 mM sodium chloride: isopropanol = 95:5 (V/V) with a flow rate of 0.5 mL/min. 50 µg of the sample was injected with an injection volume of 10 µL, and the UV absorbance at 280 nm was monitored for at least 30 min. The aggregation of the antibody-drug conjugate was calculated by the area normalization method.

Reversed-phase high performance liquid chromatography-mass spectrometry (RP-HPLC-MS) was used to detect the drug-antibody ratio (DAR for short) of the antibody-drug conjugate. The ADC sample was diluted to 1 mg/mL with 50 mmol/L Tris buffer (pH 8.0), and a freshly prepared dithiothreitol (DTT) stock solution was added to make the final concentration of DTT 50 mmol/L, followed by incubation at 37°C for 10 min. A PLRP-S column (1000 Å pore size, 2.1×50 mm, 5 µm) was used with a column temperature of 70°C. The flow rate was 0.25 mL/min. 10-20 µg of the sample was injected. Mobile phase A was an aqueous solution containing 0.1% formic acid (V/V) and 0.025% trifluoroacetic acid (V/V), and mobile phase B was an acetonitrile solution containing 0.1% formic acid (V/V) and 0.025% trifluoroacetic acid (V/V). From 0 to 3 min, 27% mobile phase B run for 3 min; from the 3 to 25 min, mobile phase B was increased from 27% to 49%; from the 25 to 26 min, mobile phase B was increased from 49% to 95%; from the 26 to 31 min, 95% mobile phase B ran; from the 30 to 31.5 min, mobile phase B was decreased from 95% to 27%; from the 31.5 to 45 min, 27% mobile phase B ran. The UV absorbance at 280 nm was monitored. The mass spectrometer parameters were as follows: the desolvation gas temperature was 350°C, the desolvation gas flow rate was 7.0 L/min, the nebulizer pressure was 40 psi, the capillary voltage was +3,000 V and -3,000 V, and the mass range was 500-1,600 m/z. Deconvolution was used to calculate the molecular weight of each mass spectrum peak (range 20,000-70,000) and compare it with the molecular weight of the unconjugated antibody heavy and light chains to calculate the drug load of each peak and determine the load of the corresponding UV peak. The weighted average DAR of the antibody-drug conjugate was calculated by integrating the area percentage of each heavy and light chain UV peak and combining the drug load distribution of each peak.

Hydrophobic interaction chromatography (HIC) was used to detect the content of naked antibody in the antibody-drug conjugate. A Biocore HIC-Butyl column (4.6×150 mm, 5 µm) was used. Mobile phase A was a 100 mM phosphate aqueous solution (pH 7.0) containing 2 M ammonium sulfate, and mobile phase B was 100 mM phosphate aqueous solution (pH 7.0): isopropanol = 80:20 (V/V). 50 µg of the sample was injected with an injection volume of 10 µL. 100% mobile phase Aran for 3 min at a flow rate of 1 mL/min. From the 3 to 25 min, mobile phase B was increased from 0% to 100%; at the 25.1 min, mobile phase B was decreased to 0% and ran until to the 30 min. The UV absorbance at 280 nm was monitored. The retention time of the naked antibody peak was located using the antibody of the antibody-drug conjugate, and the content of the naked antibody in the antibody-drug conjugate was calculated by the area normalization method.

### 2. Test results

**Table 1. Test results of ADC quality characterization**

| ADC source | ADC code | Set DAR | Actual DAR | Aggregation (%) | Naked antibody content (%) |
|---|---|---|---|---|---|
| Example 1 | SMP-656 | 4.00 | 4.01 | 0.88 | 1.89 |
| Comparative example 2 | SMP-79786-X | 4.00 | 3.65 | 2.20 | 6.39 |
| Comparative example 1 | DS-8201a | 8.00 | 8.00 | 0.40 | 0.00 |

SMP-656 and SMP-79786-X contained the same antibody and toxin, and the only difference was that SMP-656 introduced a 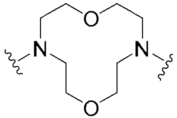 structure in the linker design. However, it can be seen from the above table that compared with SMP-79786-X, SMP-656 had lower aggregation and naked antibody content under the condition of similar DAR; in addition, under the same conjugation conditions, the actual DAR of SMP-656 was closer to the set DAR.

The above test results show that the ADC of the present disclosure had low aggregation and naked antibody content, and the DAR was basically consistent with the designed DAR, which was conducive to improving the stability of ADC, reducing its immunogenicity, reducing competitive inhibition, and improving drug efficacy.

### Test example 2: plasma stability test of antibody-drug conjugates

### 1. Test method

(1) Sample preparation: 980 µL of drug-free plasma (derived from mice, monkeys, dogs, and humans, anticoagulated with heparin sodium) was taken to pre-incubate at 37°C for 5 min, respectively. 20 µL of the ADC sample was added to the pre-incubated plasma, so that the plasma contained about 100 µg/mL of ADC, and two parallel samples were prepared. At each time point of sample incubation, 50 µL of the plasma sample was taken and added with 150 µL of ice-cold acetonitrile containing internal standard. They were mixed well by vortexing, then added with 500 µL of extraction solution (methyl tert-butyl ether-ethyl acetate = 50:50). They were mixed well further by vortexing, centrifuged at 12,000 rpm for 10 min. 600 µL of the supernatant was taken and blown dry with nitrogen, added with 100 µL of acetonitrile-water (50:50) to reconstitute, and stored at -80°C. All samples were tested together after sampling was completed.
(2) Blank sample: Drug-free plasmas from different species were incubated at 37°C. Total 50 µL drug-free plasma was taken and added with ice-cold acetonitrile without internal standard, and processed according to the sample preparation method to obtain a blank sample.
(3) Zero-concentration sample: Drug-free plasmas from different species were incubated at 37°C. Total 50 µL drug-free plasma was taken and added with ice-cold acetonitrile containing internal standard, and processed according to the sample preparation method to obtain a zero-concentration sample.
(4) Calibration curve and quality control samples: Total 20 µL of calibration standard working solution (containing small-molecule toxins to be tested 20 µg/mL-40 µg/mL) and 20 µL of quality control working solution (containing small-molecule toxins at three different concentrations: high, medium, and low, in duplicate) were added to the pre-incubated plasma, respectively. Total 50 µL of the sample was taken and added with ice-cold acetonitrile containing internal standard, and processed according to the sample preparation method to obtain calibration standard samples and quality control samples.
(5) Chromatographic conditions: A Waters Xbridge C18 column (4.6×50 mm, 3.5 µm) was used. Mobile phase A was 0.1% formic acid aqueous solution, and mobile phase B was 0.1% formic acid acetonitrile solution. From 0 to the 2.3 min, mobile phase A was decreased from 95% to 5% and maintained for 1 min; from the 3.3 to 3.4 min, mobile phase A was increased from 5% to 95% and maintained for 0.4 min. The column temperature was 40°C, the flow rate was 2 mL/min, and the injection volume was 10 µL.
(6) Mass spectrometry conditions: Positive ion selected ion monitoring (SIM) mode was employed. The target small molecule toxin was monitored at m/z 730.5 with a collision-induced dissociation (CID) energy of 90. The internal standard (IS) was monitored at m/z 787.4 with a CID energy of 120. The peak width was 0.02 min, the desolvation gas flow rate was 9 L/min, the nebulizer pressure was 60 psi, and the nebulization chamber temperature was 350°C.
(7) Data processing: A linear regression (weighting factor 1/X²) was performed with the concentration of the analyte in the calibration standards as x-axis and the peak area ratio of the analyte to the internal standard as the y-axis to calculate the concentration of the analyte in each sample solution. The percentage of the measured result relative to the theoretical content (the theoretical content was calculated based on the DAR and ADC concentration) was calculated as the payload release ratio.

### 2. Test results

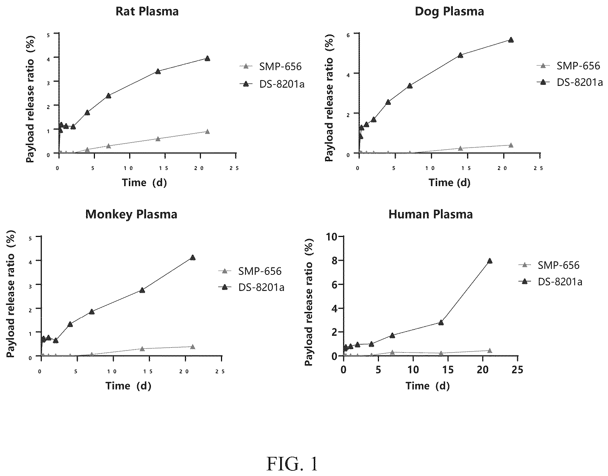

It could be seen from FIG. 1 that SMP-656 had significantly higher plasma stability than DS-8201a in mouse, dog, monkey, and human plasma.

### Test example 3: in vivo efficacy test of antibody-drug conjugates

### 1. Test method

### 1.1 Establishment of tumor models:

Tumor cells (ovarian cancer SKOV-3, gastric cancer NCI-N87, breast cancer JIMT-1, pancreatic cancer Capan-1, breast cancer HCC1954) were cultured in medium and maintained in a 37°C incubator with 5% CO₂ and saturated humidity. Tumor cells in the logarithmic growth phase were collected, resuspended in basic medium, and the cell concentration was adjusted to 8×10⁷/mL. Under sterile conditions, 0.1 mL of the cell suspension was inoculated subcutaneously into the right axilla of mice at an incubation concentration of 8×10⁶ Cells/0.1 mL/mouse.

### 1.2 Grouping and administration observation

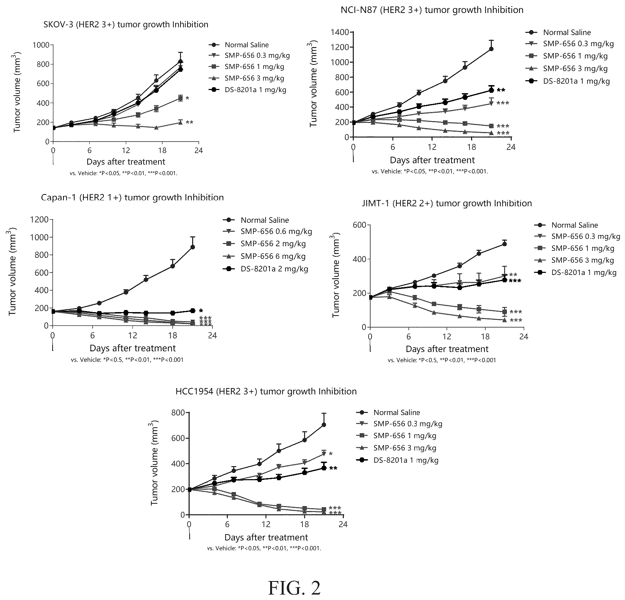

When the tumor volume reached about 100-200 mm³, the animals were randomly grouped according to the tumor volume. The grouping day was recorded as Day 0 and administration was performed according to the animal weight. The ADC administration dose was shown in FIG. 2. During the test, the animal weight and tumor volume were measured twice a week in the early stage, and the animal clinical symptoms were observed and recorded daily. The test lasts for 21 days. After the last weighing at the end of the test, the remaining animals were euthanized with CO₂, and the tumors were removed, weighed and photographed for recording.

### 2. Test results

It can be seen from FIG. 2 that in the tumor models of ovarian cancer SKOV-3, gastric cancer NCI-N87, pancreatic cancer Capan-1, breast cancer JIMT-1, and breast cancer HCC1954, SMP-656 had a significantly improved tumor-inhibiting effect at the same administration dose (1 mg/kg) compared with DS-8201a, indicating that the in vivo anti-tumor effect of SMP-656 was significantly better than that of DS-8201a.

The above test results show that the in vivo anti-tumor effect of the ADC of the present disclosure is significantly improved.

### Test example 4: in vivo efficacy duration test of antibody-drug conjugates

### 1. Test method

### 1.1 Establishment of tumor models:

Gastric cancer NCI-N87 tumor cells were cultured in medium and maintained in a 37°C incubator with 5% CO₂ and saturated humidity. Tumor cells in the logarithmic growth phase were collected, resuspended in basic medium, and the cell concentration was adjusted to 1×10⁸/mL. Under sterile conditions, 0.1 mL of cell suspension was inoculated subcutaneously into the right axilla of mice at an incubation concentration of 1×10⁷ Cells/0.1 mL/mouse.

### 1.2 Grouping and administration observation

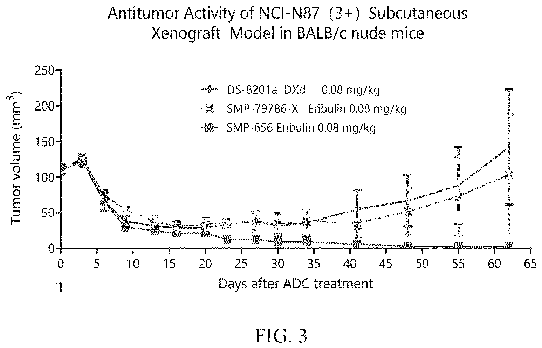

When the tumor volume reached about 110 mm³, the animals were randomly grouped according to the tumor volume. The grouping day was recorded as Day 0 and the administration was performed according to the animal weight (the administration dose based on the small-molecule toxin is shown in FIG. 3). During the test, the animal weight and tumor volume were measured twice a week in the early stage, and the animal clinical symptoms were observed and recorded daily. The test lasted for 62 days. After the last weighing at the end of the test, the remaining animals were euthanized with CO², and the tumors were removed, weighed and photographed for recording.

### 2. Test results

The test results in FIG. 3 show that under the same administration dose of small-molecule toxin and the same administration frequency, SMP-656 introduced in the linker 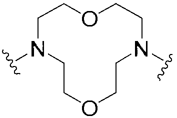 continuously inhibited tumor growth for a longer time without recurrence. However, DS-8201a and SMP-79786-X without this structure introduced in the linker began to have tumor recurrence 20 days after administration.

The above test results show that the ADC of the present disclosure has a significantly improved effect of sustained tumor inhibition in vivo and can effectively inhibit tumor recurrence.

### Test example 5: safety evaluation of antibody-drug conjugates

### 1. Test method

Four groups were set up for this test, namely the vehicle control group and SMP-656 groups with different doses (5, 10, 15 mg/kg), with 10 animals in each group, including 5 females and 5 males. Monkeys in each group were intravenously infused (for 30 min) with SMP-656 at doses of 5, 10 and 15 mg/kg at a volume of 10 mL/kg, once every three weeks, for 3 administrations (total 9 weeks), followed by a 4-week recovery period.

During the test, detailed clinical observations, and body weight and food intake monitoring were performed once a week. Clinical pathology examinations (including: hematology, coagulation, serum biochemistry, and urine analysis) were performed on Day 7, Day 50 and Day 71 respectively. The observation of irritation at the administration site was conducted once a week during the administration period, 2-4 h after administration on the administration day, and once a week during the recovery period. For animals with symptoms, the observation frequency was increased to daily until the symptoms disappeared. All surviving animals underwent accompanying toxicokinetic testing on Day 1 and Day 22. At the end of the administration period and the end of the recovery period, all surviving animals were euthanized as planned, dissected and grossly observed, and tissues and organs were weighed or fixed and preserved for pathological examination.

### 2. Test results

**Table 2. Safety evaluation results**

| | SMP-656 | MORAb-202 | Eribulin |
|---|---|---|---|
| Monkey HNSTD (mg/kg) | 10 | 6¹ | 0.03² |
| Equivalent to eribulin amount (mg/kg) | 0.20 | 0.12 | 0.03 |
| References | 1. Cancer Science. 2021;112:2467-2480 | | |
| | 2. Cancer research 61, 1013-1021, February 1, 2001 | | |

Both MORAb-202 and SMP-656 were ADCs with Eribulin as a small-molecule toxin, and both had a set DAR of 4. It can be seen from Table 2 that SMP-656 had a higher HNSTD (highest non-severe toxic dose). Based on the equivalent eribulin amount, it increased the HNSTD of eribulin by 6.7 times, proving that SMP-656 had excellent safety.

In conclusion, the present disclosure provides an antibody-drug conjugate, a preparation method, and use thereof. The antibody-drug conjugate has low aggregation and low naked antibody, appropriate DAR, exhibits excellent plasma stability and anti-tumor effect, can effectively inhibit tumor recurrence, and has a good clinical application prospect.

## Claims

1. An antibody-drug conjugate, or a stereoisomer, an optical isomer, a salt, or a deuterated derivative thereof, wherein the structure of the antibody-drug conjugate is as shown in Formula I: 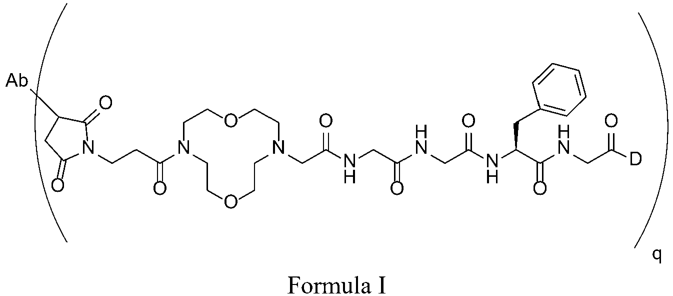
Ab is an anti-HER2 antibody, and the anti-HER2 antibody is inetetamab or trastuzumab;
q is an integer from 1 to 20; and
D is a drug.

2. The antibody-drug conjugate, or the stereoisomer, the optical isomer, the salt, or the deuterated derivative thereof according to claim 1, wherein D is a cytotoxic drug, a drug for treating autoimmune toxicity, or an anti-inflammatory drug;
preferably, D is a drug targeting DNA or a drug targeting tubulin;
more preferably, D is one of the following compounds or derivatives thereof:

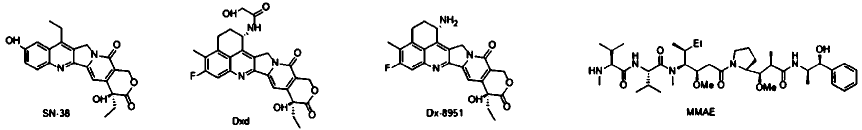

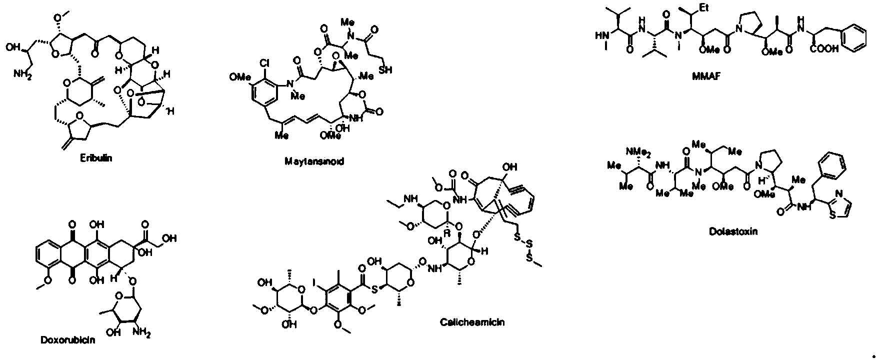

3. The antibody-drug conjugate, or the stereoisomer, the optical isomer, the salt, or the deuterated derivative thereof according to claim 2, wherein the structure of the antibody-drug conjugate is shown in Formula II: 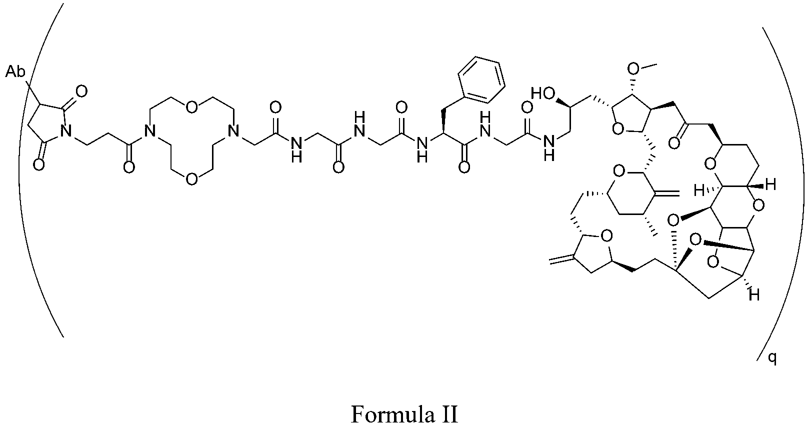
Ab is an anti-HER2 antibody, and the anti-HER2 antibody is inetetamab or trastuzumab; and
q is an integer from 1 to 20.

4. The antibody-drug conjugate, or the stereoisomer, the optical isomer, the salt, or the deuterated derivative thereof according to any one of claims 1-3, wherein the heavy chain sequence of the inetetamab is as shown in SEQ ID NO: 1, and the light chain sequence is shown in SEQ ID NO: 2.

5. The antibody-drug conjugate, or the stereoisomer, the optical isomer, the salt, or the deuterated derivative thereof according to any one of claims 1-3, wherein the heavy chain sequence of the trastuzumab is as shown in SEQ ID NO: 3, and the light chain sequence is shown in SEQ ID NO: 4.

6. The antibody-drug conjugate, or the stereoisomer, the optical isomer, the salt, or the deuterated derivative thereof according to any one of claims 1-3, wherein the DAR of the antibody-drug conjugate is 1.00-20.00, preferably 2.0-8.0.

7. The antibody-drug conjugate, or the stereoisomer, the optical isomer, the salt, or the deuterated derivative thereof according to claim 6, wherein the DAR of the antibody-drug conjugate is 2.0-5.0, preferably 4.08±0.5.

8. A pharmaceutical preparation for preventing and/or treating tumors, wherein it is prepared by using the antibody-drug conjugate, or the stereoisomer, the optical isomer, the salt, or the deuterated derivative thereof according to any one of claims 1-7 as an active ingredient, together with pharmaceutically acceptable excipients.

9. The pharmaceutical preparation according to claim 8, wherein the preparation is an oral preparation or an injection preparation.

10. Use of the antibody-drug conjugate, or the stereoisomer, the optical isomer, the salt, or the deuterated derivative thereof according to any one of claims 1-7 in the preparation of a pharmaceutical preparation for preventing and/or treating tumors.

11. The use according to claim 10, wherein the tumor is selected from lung cancer, urethral cancer, colorectal cancer, prostate adenocarcinoma, ovarian cancer, pancreatic cancer, breast cancer, bladder cancer, gastric cancer, gastrointestinal stromal tumor, cervical cancer, esophageal cancer, squamous cell carcinoma, peritoneal cancer, liver cancer, colon cancer, rectal cancer, colorectal cancer, uterine cancer, salivary gland cancer, kidney cancer, vulvar cancer, thyroid cancer, penile cancer, leukemia, malignant lymphoma, plasmacytoma, myeloma, or sarcoma, and biliary tract tumor.

12. A method for preparing the antibody-drug conjugate, or the stereoisomer, the optical isomer, the salt, or the deuterated derivative thereof according to any one of claims 1-7, wherein the method comprises the following steps: conjugating an anti-HER2 antibody with Compound III to obtain the antibody-drug conjugate; the structure of Compound III is as follows: 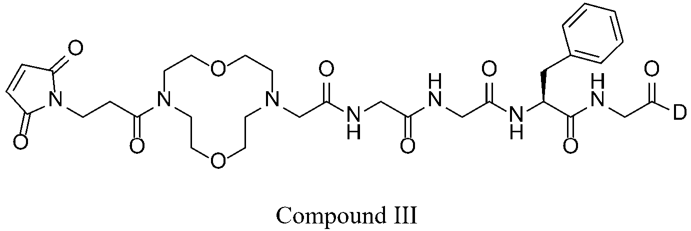 D is the drug described in any one of claims 1-7.
